# EUROPEAN PATENT APPLICATION

(11) **EP 4 083 056 A1**
(43) Date of publication of application: **02.11.2022**
(21) Application number: 20908279.1
(22) Date of filing: 15.12.2020
(51) Int. Cl.: C07K 14/415, A23L 2/00, A23L 2/66

(54) **NON-ALCOHOLIC BEER-FLAVORED BEVERAGE, RICH TASTE ENHANCING AGENT AND SOURNESS REDUCING AGENT FOR NON-ALCOHOLIC BEER-FLAVORED BEVERAGES, AND RICH TASTE ENHANCING METHOD AND SOURNESS REDUCING METHOD FOR NON-ALCOHOLIC BEER-FLAVORED BEVERAGES**

(30) Priority: 27.12.2019 JP 2019239771
(71) Applicant: Suntory Holdings Limited, Osaka 530-8203 (JP)
(72) Inventor: IWASA, Keiko, Soraku-gun, Kyoto 619-0284 (JP); BEPPU, Yoshinori, Soraku-gun, Kyoto 619-0284 (JP); MATSUO, Yoshihide, Soraku-gun, Kyoto 619-0284 (JP); FUJITA, Yohei, Mishima-gun, Osaka 618-0001 (JP)
(74) Representative: Vossius & Partner Patentanwälte Rechtsanwälte mbB
(86) International application number: PCT/JP2020/046834
(87) International publication number: WO 2021/131924

(57) **Abstract**

The present invention aims to provide a non-alcoholic beer-taste beverage with an enhanced rich flavor and a reduced sour flavor using a grain-derived raw material. The present invention relates to a non-alcoholic beer-taste beverage having an alcohol content of less than 1.0%, the non-alcoholic beer-taste beverage containing a glycated protein which is a grain-derived protein, has a glycated amino group of lysine, and has a molecular weight of 35 to 50 kDa.

## Description

### TECHNICAL FIELD

The present invention relates to a non-alcoholic beer-taste beverage, a rich flavor enhancing agent and a sour flavor reducing agent for non-alcoholic beer-taste beverages, and a rich flavor enhancing method and a sour flavor reducing method for non-alcoholic beer-taste beverages.

### BACKGROUND ART

Diversification of consumer preferences in recent years has created a desire for development of non-alcoholic beer-taste beverages having various aroma and taste characteristics.

For example, Patent Literature 1 discloses use of a sweet component, an aldehyde-based component, or the like as a flavor improver for beer-like beverages in order to enhance the rich taste and the like of beer-like beverages.

### CITATION LIST

### - Patent Literature

Patent Literature 1: JP 2016-214262 A

### SUMMARY OF INVENTION

### - Technical Problem

However, the flavor improving agents as in Patent Literature 1 affect the aroma and taste and may not be suitable for use in some cases.

Raw materials derived from a grain such as wheat or corn are commonly used as raw materials for non-alcoholic beer-taste beverages. Since the grain-derived raw materials are commonly used for non-alcoholic beer-taste beverages, the manufacturers and beverage consumers will feel comfortable about use of a grain-derived raw material as an ingredient for enhancing the rich flavor. Thus, a method has been desired which enhances the rich flavor using a grain-derived raw material.

In addition, an acid such as citric acid may be added to a non-alcoholic beer-taste beverage in order to lower the pH and thus prevent spoilage of the beverage. Addition of an acid, however, adds a sour flavor to the beverage. Thus, a method has been desired which reduces the sour flavor while keeping the same pH.

The present invention aims to provide a non-alcoholic beer-taste beverage with an enhanced rich flavor and a reduced sour flavor using a grain-derived raw material. The present invention also aims to provide a rich flavor enhancing agent for non-alcoholic beer-taste beverages and a sour flavor reducing agent for non-alcoholic beer-taste beverages, each of the agents being produced from a grain-derived raw material. Furthermore, the present invention aims to provide a rich flavor enhancing method for non-alcoholic beer-taste beverages and a sour flavor reducing method for non-alcoholic beer-taste beverages, each of the methods using a grain-derived raw material.

### - Solution to Problem

In other words, the present invention relates to the following non-alcoholic beer-taste beverage and the like.
(1) A non-alcoholic beer-taste beverage having an alcohol content of less than 1.0%, the non-alcoholic beer-taste beverage containing a glycated protein which is a grain-derived protein, has a glycated amino group of lysine, and has a molecular weight of 35 to 50 kDa.
(2) The non-alcoholic beer-taste beverage according to (1) above, wherein the glycated protein has a glycated lysine content of 4.7 nmol/mL or more.
(3) The non-alcoholic beer-taste beverage according to (1) or (2) above, wherein the glycated protein has a glycated lysine content of 9.4 nmol/mL or more.
(4) The non-alcoholic beer-taste beverage according to any one of (1) to (3) above, wherein a sugar bound to the glycated amino group of the lysine is a monosaccharide or a disaccharide.
(5) The non-alcoholic beer-taste beverage according to any one of (1) to (4) above, wherein the grain is at least one selected from the group consisting of barley, wheat, corn, rice, and soybean.
(6) The non-alcoholic beer-taste beverage according to any one of (1) to (5) above, wherein the non-alcoholic beer-taste beverage contains no wort.
(7) A rich flavor enhancing agent for non-alcoholic beer-taste beverages having an alcohol content of less than 1.0%, the agent containing a glycated protein which is a grain-derived protein, has a glycated amino group of lysine, and has a molecular weight of 35 to 50 kDa.
(8) A rich flavor enhancing method for non-alcoholic beer-taste beverages, the method including enhancing a rich flavor of a non-alcoholic beer-taste beverage by adding a glycated protein which is a grain-derived protein, has a glycated amino group of lysine, and has a molecular weight of 35 to 50 kDa to the non-alcoholic beer-taste beverage having an alcohol content of less than 1.0%.
(9) A sour flavor reducing agent for non-alcoholic beer-taste beverages having an alcohol content of less than 1.0%, the agent containing a glycated protein which is a grain-derived protein, has a glycated amino group of lysine, and has a molecular weight of 35 to 50 kDa.
(10) A sour flavor reducing method for non-alcoholic beer-taste beverages, the method including reducing a sour flavor of a non-alcoholic beer-taste beverage by adding a glycated protein which is a grain-derived protein, has a glycated amino group of lysine, and has a molecular weight of 35 to 50 kDa to the non-alcoholic beer-taste beverage having an alcohol content of less than 1.0%.

### - Advantageous Effects of Invention

The present invention can provide a non-alcoholic beer-taste beverage with an enhanced rich flavor and a reduced sour flavor using a grain-derived raw material. The present invention can also provide a rich flavor enhancing agent for non-alcoholic beer-taste beverages and a sour flavor reducing agent for non-alcoholic beer-taste beverages, each of the agents being produced from a grain-derived raw material. Furthermore, the present invention can provide a rich flavor enhancing method for non-alcoholic beer-taste beverages and a sour flavor reducing method for non-alcoholic beer-taste beverages, each of the methods using a grain-derived raw material.

### DESCRIPTION OF EMBODIMENTS

The non-alcoholic beer-taste beverage of the present invention contains a glycated protein.

The glycated protein has a molecular weight of 35 to 50 kDa.

Proteins having a molecular weight of 35 to 50 kDa are those showing a band pattern between 35 to 50 kDa in electrophoresis by SDS-PAGE after ultrafiltration of a raw material solution of a non-alcoholic beer-taste beverage using a 30 kDa cutoff membrane.

The proteins are preferably those having a molecular weight of about 40 kDa. Herein, such proteins having a molecular weight of about 40 kDa are also referred to as 40 kDa proteins.

The glycated protein is a grain-derived protein.

The grain is preferably at least one selected from the group consisting of barley, wheat, corn, rice, and soybean.

When the grain is a cereal grain, the raw non-alcoholic beer-taste beverage can contain a protein derived from a known cereal grain usable in production of a non-alcoholic beer-taste beverage. Examples of the cereal grain include barley, wheat, rye, common wild oat *(Avena fatua*), and common oat *(Avena sativa*). Preferred is barley. Further, the cereal grain may be either germinated or ungerminated, but is preferably malt from a germinated cereal grain. The non-alcoholic beer-taste beverage may contain protein(s) from any one of these cereal grains alone or proteins from any two or more of these cereal grains in combination.

The glycated protein is a grain-derived protein which has a glycated amino group of lysine.

The phrase "having a glycated amino group of lysine" herein means that a sugar is bound to an amino group of lysine.

The sugar is preferably bound to a side-chain amino group among the amino groups of lysine.

The glycation of an amino group of lysine can be confirmed by colorimetric quantification by the nitroblue tetrazolium (NBT) method.

The sugar bound to the glycated amino group of lysine may be a monosaccharide, a disaccharide, or a trisaccharide or higher polysaccharide, but is preferably a monosaccharide or a disaccharide.

Examples of the monosaccharide include all sugars including glucose, fructose, galactose, mannose, and arabinose.

Examples of the disaccharide include maltose-type disaccharides such as maltose and lactose.

Examples of the trisaccharide include maltotriose, sertriose, fucosyl lactose, and sialyllactose.

Containing the glycated protein as described above, the non-alcoholic beer-taste beverage of the present invention can be a non-alcoholic beer-taste beverage with an enhanced rich flavor and a reduced sour flavor.

Also, since the glycated protein is a grain-derived raw material, the glycated protein is prevented from having an unintentional influence on the aroma and taste unlike the flavor improving agent described in Patent Literature 1. In addition, since the grain-derived raw material is commonly used for non-alcoholic beer-taste beverages, the manufacturers and beverage consumers will feel comfortable about use of the grain-derived raw material as an ingredient for enhancing the rich flavor or for reducing the sour flavor.

The "rich flavor" as used herein is a taste that cannot be expressed by the five basic tastes, i.e., sweet, salty, sour, bitter, and umami. It is the taste expressed by the intensity of the taste (powerfulness), spread of taste, thickness of the taste, changes in taste over time (lingering taste or persistence of the taste), and the like. The phrase "enhancing the rich flavor" herein includes, for example, imparting a rich flavor to a food or beverage no having a rich flavor and enhancing the rich flavor of a food or beverage having a rich flavor. The presence or degree of the rich flavor can be evaluated by sensory evaluation by a specialist panel.

The presence or degree of the sour flavor can also be evaluated by sensory evaluation by a specialist panel.

The non-alcoholic beer-taste beverage of the present invention has a glycated lysine content of preferably 4.7 nmol/mL or more for reduction of the sour flavor of the non-alcoholic beer-taste beverage.

Further, the non-alcoholic beer-taste beverage has a glycated lysine content of preferably 9.4 nmol/mL or more for enhancement of the rich flavor of the non-alcoholic beer-taste beverage.

The non-alcoholic beer-taste beverage has a glycated lysine content of also preferably 50.0 nmol/mL or more, preferably 80.0 nmol/mL or more, preferably 100 nmol/mL or more.

The non-alcoholic beer-taste beverage also has a glycated lysine content of preferably 50.0 to 1000 nmol/mL, preferably 80.0 to 1000 nmol/mL, preferably 100 to 1000 nmol/mL.

The glycated lysine content can be quantified by colorimetric quantification by the NBT method.

The non-alcoholic beer-taste beverage of the present invention may contain any amount of the extract, but preferably contains 0.01 to 20.0% by weight of the extract.

The non-alcoholic beer-taste beverage of the present invention may be produced using malt as a raw material.

The proportion of malt in the raw material may be 0% by weight.

Here, the "proportion of malt" refers to the proportion by weight of malt in a mixture of raw materials excluding water and hops but including, for example, malt, rice, corn, sorghum, potato, starch, a cereal grain(s) other than the malt, and sugars. Here, ingredients that can be added in trace amounts, such as acidulants, sweeteners, bittering agents, seasonings, and flavorings, are not included in calculation of the proportion above.

The non-alcoholic beer-taste beverage of the present invention may not contain wort.

The non-alcoholic beer-taste beverage of the present invention has a purine concentration of preferably less than 5.0 mg/100 mL. The non-alcoholic beer-taste beverage also preferably has a purine concentration of 0 mg/100 mL.

Adding a glycated protein to a non-alcoholic beer-taste beverage having a purine concentration falling within any of the ranges above especially enhances the rich flavor and reduces the sour flavor of the beverage.

The non-alcoholic beer-taste beverage is a beer-taste beverage having an alcohol content of less than 1%, preferably one containing substantially no alcohol. The alcohol content may be 0%.

The beer-taste beverage is a beer-flavored carbonated beverage.

Beverages containing substantially no alcohol include beverages containing an undetectable trace amount of alcohol. Beverages having an alcohol content rounded to 0.0%, particularly those having an alcohol content rounded to 0.00%, are included in non-alcoholic beer-taste beverages. Examples of the non-alcoholic beer-taste beverage of the present invention include non-alcoholic beer-taste beverages and beer-taste soft drinks. The "alcohol content" here means the ethanol content, with fatty alcohols being excluded.

The alcohol content of the non-alcoholic beer-taste beverage of the present invention means the proportion (v/v%) of the alcohol in the beverage, and can be measured by any known method such as a vibrating density meter. Specifically, a beverage is filtered or sonicated to prepare a sample from which carbon dioxide has been removed. The sample is distilled over open fire to obtain a distillate. The density of the distillate at 15°C is measured. The density is then converted to the alcohol content using an appendix "Table 2: Table of Conversion between Alcohol Content and Density (15°C) or Specific Gravity (15/15°C)" of the Official Analysis Method of National Tax Agency of Japan (National Tax Agency Directive No. 6 in 2007, revised on June 22, 2007). When the alcohol content is less than 1.0%, a commercially available alcohol measuring instrument or a gas chromatograph may be used.

A common process of producing a non-alcoholic beer-taste beverage is described below.

With no fermentation step with yeast, a non-alcoholic beer-taste beverage can be easily produced. Common nonfermented non-alcoholic beer-taste beverages include those produced using malt as a raw material and those not, and can be produced as described below.

In production of non-alcoholic beer-taste beverages produced using malt as a raw material, a mixture containing water and raw materials, such as a cereal grain (e.g., a malted grain), and other grains, starch, sugars, bittering agents, or colorants as needed, is mixed with an enzyme such as an amylase as needed. The resulting mixture is gelatinized, saccharified, and filtered to obtain a saccharified solution. The saccharified solution is mixed with hops, a bittering agent, or the like as needed and then boiled, followed by removal of the solids content such as a coagulated protein in a clarification tank. The saccharified solution may be replaced by a boiled mixture of a malt extract, warm water, and hops. Hops may be added to the mixture at any stage from the start of boiling to the end of boiling. The conditions in the saccharification, boiling, solids content removal, and the like may be known conditions. After the boiling, the obtained wort is filtered, and the filtrate is then mixed with carbon dioxide gas. Thereafter, the resulting mixture is packed into a container and sterilized to obtain a desired non-alcoholic beer-taste beverage. When a glycated protein is to be added in any of the steps above, the glycated protein may be added in any of the steps before packing.

In production of a non-alcoholic beer-taste beverage not using malt as a raw material, a liquid sugar containing a carbon source, a nitrogen source as an amino acidcontaining material other than a cereal grain or malt, hops, colorants, and the like are mixed together with warm water to obtain a liquid sugar solution. The liquid sugar solution is boiled. When hops are used as a raw material, the hops may be mixed into the liquid sugar solution during boiling, not before the start of boiling. The boiled liquid sugar solution is mixed with a carbon dioxide gas. Thereafter, the resulting mixture is packed into a container and sterilized to obtain a desired non-alcoholic beer-taste beverage. When a glycated protein is to be added in any of the steps above, the glycated protein may be added in any of the steps before packing.

An aliphatic alcohol may be added to the non-alcoholic beer-taste beverage of the present invention in order to impart the alcohol-like texture to the beverage. The aliphatic alcohol may be any known aliphatic alcohol, but is preferably a C4-C5 aliphatic alcohol. Preferred aliphatic alcohols in the present invention include 2-methyl-1-propanol and 1-butanol as C4 aliphatic alcohols and 3-methyl-1-butanol, 1-pentanol, and 2-pentanol as C5 aliphatic alcohols. These can be used alone or in combination of two or more thereof.

The C4-C5 aliphatic alcohol content is preferably 0.0002 to 0.0007% by mass, more preferably 0.0003 to 0.0006% by mass. Herein, the aliphatic alcohol content can be measured by headspace gas chromatography.

The non-alcoholic beer-taste beverage of the present invention is preferably low in calories to suit the recent preference for low-calorie products. The non-alcoholic beer-taste beverage of the present invention therefore has a calorie content of preferably less than 5 kcal/100 mL, more preferably less than 4 kcal/100 mL, still more preferably less than 3 kcal/100 mL.

The calorie content of the non-alcoholic beer-taste beverage of the present invention is basically calculated in accordance with "Method for Analysis of Components such as Nutritional Components and the like under the Nutrition Labeling Standards" which was published in connection with the Health Promotion Act in Japan. In other words, in principle, the calorie content can be calculated as a sum of the products of the quantified amounts of the nutritional components multiplied by the energy conversion coefficients of the respective components (protein: 4 kcal/g, fat: 9 kcal/g, sugar: 4 kcal/g, dietary fiber: 2 kcal/g, alcohol: 7 kcal/g, organic acid: 3 kcal/g). The details are described in "Method for Analysis of Components such as Nutritional Components and the like under the Nutrition Labeling Standards".

The specific method of measuring the amount of each nutritional component contained in the non-alcoholic beer-taste beverage of the present invention may be the corresponding analytical method described in "Method for Analysis of Components such as Nutritional Components and the like under the Nutrition Labeling Standards" of the Health Promotion Act. Alternatively, Japan Food Research Laboratories will be available to determine the calorie contents and/or the amounts of nutritional components.

The sugar contained in the non-alcoholic beer-taste beverage of the present invention means a sugar based on the Nutrition Labeling Standards for Foods (Ministry of Health, Labor and Welfare, Notification No. 176, 2003). Specifically, the sugar refers to a component which remains after the protein, fat, dietary fiber, ash content, alcohol content, and moisture content are removed from a food. The amount of sugar in a food is calculated by subtracting the amounts of protein, fat, dietary fiber, ash content, and moisture content from the weight of the food. In this case, the amounts of protein, fat, dietary fiber, ash content, and moisture content are measured by the methods under the Nutrition Labeling Standards. Specifically, the amount of protein is measured by the nitrogen quantification conversion method. The amount of fat is measured by an ether extraction method, a chloroformmethanol liquid mixture extraction method, the Gerber method, an acid decomposition method, or the Roese-Gottlieb method. The amount of dietary fiber is measured by high performance liquid chromatography or the Prosky method. The amount of ash content is measured by a method of ashing with magnesium acetate, a direct ashing method, or a method of ashing with sulfuric acid. The amount of moisture content is measured by the Karl Fischer method, a method using a drying aid, a vacuum thermal drying method, an atmospheric thermal drying method, or a plastic film method.

The non-alcoholic beer-taste beverage of the present invention may be low in sugars to suit the recent preference for low-sugar food and beverages. The non-alcoholic beer-taste beverage of the present invention may have a sugar content of less than 2.5 g/100 mL or less than 0.5 g/100 mL. The lower limit is not particularly set, but is usually about 0.1 g/100 mL and may be, for example, 0.15 g/100 mL or more, or 0.2 g/100 mL or more.

The non-alcoholic beer-taste beverage of the present invention may contain an acidulant. The acidulant is preferably at least one acid selected from the group consisting of citric acid, lactic acid, phosphoric acid, and malic acid. In the present invention, an acid other than the acids above, such as succinic acid, tartaric acid, fumaric acid, or glacial acetic acid may also be used. Any of these can be used without limitation as long as they are approved to be added to foods. In the present invention, preferred is a combination of lactic acid, which appropriately imparts a mild sour flavor, and phosphoric acid, which appropriately imparts a slightly tingling sour flavor.

The acidulant content in the non-alcoholic beer-taste beverage of the present invention in citric acid equivalent is preferably 200 ppm or more, more preferably 550 ppm or more, still more preferably 700 ppm or more in order to impart the beer-like taste. The acidulant content is preferably 15000 ppm or less, more preferably 5500 ppm or less, still more preferably 2000 ppm or less in terms of sour flavor. The acidulant content in the present invention in citric acid equivalent therefore falls within a range of preferably 200 ppm to 15000 ppm, more preferably 550 ppm to 5500 ppm, still more preferably 700 ppm to 2000 ppm. The "acidulant content in citric acid equivalent" is an amount calculated from the degree of the sour flavor of each acidulant based on the degree of the sour flavor of citric acid. For example, a lactic acid content of 100 ppm corresponds to a citric acid equivalent of 120 ppm. A phosphoric acid content of 100 ppm corresponds to a citric acid equivalent of 200 ppm. A maleic acid content of 100 ppm corresponds to a citric acid equivalent of 125 ppm.

The acidulant content in a non-alcoholic beer-taste beverage refers to one calculated based on analysis by high performance liquid chromatography (HPLC) or the like.

In the non-alcoholic beer-taste beverage of the present invention, hops can be used as one of the raw materials.

When hops are used, hop pellets, hop powder, and hop extracts usually used in production of beer and the like can be appropriately selected according to the desired aroma and taste. Also, processed hop products such as isometric hops and reduced hops may be used. The hops used in the non-alcoholic beer-taste beverage of the present invention include these hops. The amount of hops to be added is not particularly limited, but is typically about 0.0001 to 1% by weight based on the total amount of the beverage.

The non-alcoholic beer-taste beverage of the present invention may contain any other raw materials as needed, as long as the effect(s) of the present invention is/are not impaired. For example, sweeteners (including highintensity sweeteners), bittering agents, flavorings, yeast extracts, colorants such as caramel color, plant-extracted saponin-based substances such as soybean saponin and quillaja saponin, substances containing proteins and peptides from plants such as corn and soybean, proteinbased substances such as bovine serum albumin, seasonings such as dietary fiber and amino acids, and antioxidants such as ascorbic acid can be used as needed as long as the effect(s) of the present invention is/are not impaired.

The non-alcoholic beer-taste beverage of the present invention has a pH of preferably 3.0 to 5.0, more preferably 3.5 to 4.5, still more preferably 3.5 to 4.0, in order to improve the flavor of the beverage.

The non-alcoholic beer-taste beverage of the present invention can be packed in a container. The form of the container is not limited. The non-alcoholic beer-taste beverage can be packed in a sealed container such as a bottle, can, keg, or PET bottle, whereby a packed beverage can be obtained.

The rich flavor enhancing agent of the present invention is a rich flavor enhancing agent for non-alcoholic beer-taste beverages having an alcohol content of less than 1.0%, the agent containing a glycated protein which is a grain-derived protein, has a glycated amino group of lysine, and has a molecular weight of 35 to 50 kDa.

Also, the sour flavor reducing agent of the present invention is a sour flavor reducing agent for non-alcoholic beer-taste beverages having an alcohol content of less than 1.0%, the agent containing a glycated protein which is a grain-derived protein, has a glycated amino group of lysine, and has a molecular weight of 35 to 50 kDa.

An agent containing a glycated protein which is a grain-derived protein, has a glycated amino group of lysine, and has a molecular weight of 35 to 50 kDa can be an agent that functions simultaneously as a rich flavor enhancing agent and a sour flavor reducing agent for non-alcoholic beer-taste beverages.

Hereinafter, the rich flavor enhancing agent and the sour flavor reducing agent for non-alcoholic beer-taste beverages are collectively described.

The rich flavor enhancing agent and the sour flavor reducing agent of the present invention are a glycated protein itself or a composition containing a glycated protein which are used to enhance the rich flavor of a non-alcoholic beer-taste beverage and/or to reduce the sour flavor of a non-alcoholic beer-taste beverage.

The glycated protein is a grain-derived protein, has a glycated amino group of lysine, and has a molecular weight of 35 to 50 kDa, and the details thereof are as described above.

When the rich flavor enhancing agent and the sour flavor reducing agent are a composition containing the glycated protein, they may contain known additives that can be added to a beverage as long as the effect(s) of the present invention is/are not impaired.

The glycated protein in each of the rich flavor enhancing agent and the sour flavor reducing agent of the present invention has a glycated lysine content (nmol) per microgram of the glycated protein of preferably 0.04 nmol/µg or more, more preferably 0.4 nmol/µg or more.

Since such a glycated protein has a high glycated lysine content, it is usable, even in a small amount, as a rich flavor enhancing agent capable of enhancing the rich flavor of a non-alcoholic beer-taste beverage or as a sour flavor reducing agent for reducing the sour flavor of a non-alcoholic beer-taste beverage.

The rich flavor enhancing agent and the sour flavor reducing agent of the present invention can be obtained by purifying a glycated protein being a grain-derived protein, having a glycated amino group of lysine, and having a molecular weight of 35 to 50 kDa from beer that was brewed from North American malt and contains a large amount of the glycated protein.

The rich flavor enhancing agent and the sour flavor reducing agent of the present invention can also be obtained by purifying a glycated protein being a grain-derived protein, having a glycated amino group of lysine, and having a molecular weight of 35 to 50 kDa from malt or barley containing the glycated protein.

Also, mashing may be performed to extract proteins such that they include a large amount of a glycated protein having a glycated amino group of lysine and having a molecular weight of 35 to 50 kDa.

The method of producing a non-alcoholic beer-taste beverage of the present invention is not particularly limited. Examples of the method include a production method including adding a glycated protein having a molecular weight of 35 to 50 kDa to a non-alcoholic beer-taste beverage having an alcohol content of less than 1.0%.

Specific examples of the production method include a method of producing a non-alcoholic beer-taste beverage with an enhanced rich flavor and/or a reduced sour flavor, the method including adding a glycated protein which is a grain-derived protein, has a glycated amino group of lysine, and has a molecular weight of 35 to 50 kDa to the non-alcoholic beer-taste beverage having an alcohol content of less than 1.0%.

The method of producing a non-alcoholic beer-taste beverage by the method above is also a rich flavor enhancing method for the non-alcoholic beer-taste beverage of the present invention and a sour flavor reducing method for the non-alcoholic beer-taste beverage of the present invention.

When the rich flavor enhancing agent or the sour flavor reducing agent is added to a non-alcoholic beer-taste beverage, the amount of each of the rich flavor enhancing agent and the sour flavor reducing agent to be added is determined such that the glycated lysine content in the glycated protein is preferably 9.4 nmol/mL or more for enhancement of the rich flavor, and is preferably 4.7 nmol/mL or more for reduction of the sour flavor.

It is also preferred that the glycated lysine content in the glycated protein is 50.0 nmol/mL or more, 80.0 nmol/mL or more, or 100 nmol/mL or more.

In this case, the amount of each of the rich flavor enhancing agent and the sour flavor reducing agent to be added is determined such that the amount of the glycated protein to be added is preferably 5 µg/mL or more for enhancement of the rich flavor, and is preferably 10 µg/mL or more for reduction of the sour flavor.

### EXAMPLES

### (Purification of beer-derived glycated protein)

North American malt was crushed to an appropriate particle size, introduced into a mashing tank, and then mixed with warm water. The mixture was kept at a temperature suitable for a malt enzyme for a period of time sufficient for saccharification to convert starch into a sugar by the action of the malt enzyme, whereby a saccharified liquid was produced. Subsequently, the saccharified liquid was filtered, and hop was added to the resulting filtrate, followed by boiling, whereby hot wort was produced for use for fermentation. The hot wort was cooled, and beer yeast was added thereto. The sugar in the wort was broken down into alcohol and carbon dioxide by the action of the yeast during a few days, whereby newly fermented beer was produced. The newly fermented beer was transferred to a beer storage tank, and stored at a low temperature for several tens of days. During this period, the beer was aged, and the taste and aroma of the beer were harmonized. After aging, the beer was filtered and bottled.

A glycated protein was purified as described below from the beer brewed from North American malt by the method described above.

### (1) Fractionation by cation exchange resin

A cation-exchange resin "SP Sepharose" (50 mL) was placed in an empty column. Beer brewed from North American malt was adsorbed onto the resin. Subsequently, the resin used for adsorption was transferred to another column, washed with a 20 mM sodium acetate buffer (pH 4.5), and then eluted with 20 mM sodium acetate (pH 4.5) + 0.5 M-NaCl, whereby fractions were collected. The resulting fractions were evaluated by SDS-PAGE, and fractions containing a 40 kDa glycated protein were collected as cation-exchange resin-bound fractions.

### (2) Ultrafiltration (buffer exchange)

The cation-exchange resin-bound fractions obtained in (1) above were added to an ultrafiltration unit (Amicon Ultra-15 30K available from Merck KGaA) washed with water, centrifuged at 3500 rpm and ultrafiltrated, whereby a concentrate was obtained.

### (3) Ammonium sulfate fraction

The concentrate obtained in (2) above was dropped into a beaker charged with a 20 mM phosphate buffer (pH 7.0) and 2 M ammonium sulfate, followed by stirring. The resulting suspension was then centrifuged (2330 g, 10 minutes, room temperature). The supernatant was collected in a different container. The collected solution was concentrated using an ultrafiltration unit. To the concentrate was added 20 mM sodium acetate (pH 4.5), followed by centrifugation (2330 g, 10 minutes, room temperature) for concentration, so that a purified glycated protein product (quantified by the Bradford assay (in bovine serum albumin (BSA) equivalent), 20.4 mg/mL, 2.21 mL) was obtained. The purity of the resulting purified glycated protein product was confirmed by SDS-PAGE.

### (Modification analysis of glycated protein)

### (1) Preparation of gel pieces

An amount of 10 µL of a solution of the purified glycated proteins (concentration 1 mg/mL, 20 mM sodium acetate buffer (pH 4.5)) was electrophoresed on SDS-PAGE, stained with Coomassie brilliant blue (CBB), and then a gel of glycated proteins giving a band around 40 kDa was cut out.

### (2) Enzymatic digestion of protein

The cut-out gel piece was sliced, followed by reduction with dithiothreitol (56°C, 1 hr) and carbamide methylation with iodoacetamide (room temperature under light-shielded conditions, 45 min). Then, a 0.01% ProteaseMax-containing 10 ng/µL chymotrypsin solution (5 mM calcium chloride, 50 mM ammonium bicarbonate solution) (15 µL), 5 mM calcium chloride, and a 50 mM ammonium bicarbonate solution (15 µL) were added, followed by overnight incubation. Subsequently, the resulting enzyme digestion solution was collected. The collected solution was solidified by drying in vacuum, which was then redissolved in a 0.1% formic acid solution.

### (3) Measurement by LC-MS/MS

LC-MS/MS measurement was performed under the following conditions.
Device used: direct flow type nano LC system "Easy-nLC 1000^{™}" (Thermo Fisher Scientific)
Trap column: Acclaim PepMap^{®} (Thermo Fisher Scientific) Analysis column: Nano HPLC Capillary Column (Nikkyo Technos Co., Ltd.)
Liquid chromatograph mass spectrometer: Q Exactive Plus (Thermo Fisher Scientific)
Mobile phase: solvent A: 0.1% formic acid/water; solvent B: 0.1% formic acid/acetonitrile
Flow rate: 300 nL/min
Gradient: 0-40% B/0-30 min, 40-60% B/30-35 min, 60-90% B/35-37 min, 90% B/37-45 min
Amount introduced: 10 µL
Ionization mode: ESI Positive
Measurement range: MS1 (m/z 350-1750)
Data dependent scan mode

### (4) Analysis of protein and modification

The protein was identified and modification was searched under the following conditions.
Search software: Proteome Discoverer 2.2.0.388 (available from Thermo Fisher Scientific)
Species: barley *(Hordeum vulgare),* hop *(Humulus),* yeast *(Saccharomyces cerevisiae)*
Search conditions: digestive enzyme: Chymotrypsin
Modification (Static): Carbamidomethyl (Cysteine)
Modification (Dynamic): Oxidation (Methionine), Hex (K, R, Protein N-term), Hex(2) (K, R, Protein N-term), Hex(3) (K, R, Protein N-term), Acetyl (Protein N-term)
Precursor ion mass error range: Monoisotopic, ± 10 ppm
Product ion mass error range: ± 0.02 Da
Maximum number of missed cleavages: 5
Confidence level (Percolator): High (level with the highest confidence of the three levels of confidence)
Database: SwissProt

The modification analysis confirmed that amino groups of lysine in the glycated proteins were glycated.

### (Evaluation of sugar modification degree of glycated proteins)

The sugar modification degree of the purified glycated proteins was colorimetrically quantified by the NBT method using a Fructosamine Assay kit (available from Abcam Plc, ab228558).

The sugar modification degree of the purified glycated proteins derived from beer was determined.

The number of tests for each protein was set to n = 2, and the average value thereof was used.

The glycated lysine content per microgram of a glycated protein was 0.94 nmol/µg (glycated protein).

### (Measurement of concentration of glycated protein in commercially available non-alcoholic beer-taste beverages)

The concentrations of glycated proteins in commercially available non-alcoholic beer-taste beverages A and B were determined (n = 2) with the LabChip^{™} GXII system (available from PerkinElmer Co., Ltd.) using HT Protein Express Chips in conformity with the standard protocol. The system detected no glycated proteins in the commercially available non-alcoholic beer-taste beverage A and the commercially available non-alcoholic beer-taste beverage B.

### (Evaluation of sour flavor and rich flavor of commercially available non-alcoholic beer taste beverage A to which glycated proteins were added)

The purified glycated proteins derived from beer were added to the commercially available non-alcoholic beer-taste beverage A, for sensory evaluation of the sour flavor and the rich flavor.

The commercially available non-alcoholic beer taste beverage A has an alcohol content of 0.00%.

The commercially available non-alcoholic beer taste beverage A has a purine concentration of less than 5.0 mg/100 mL. The total purine concentration was determined by the degradation method by the Japan Food Research Laboratories (the same applies hereinafter).

In the sensory evaluation, five special panelists rated the commercially available non-alcoholic beer-taste beverage A containing the purified glycated proteins in 0.1-point increments, with a reference point of 3.0 for the sour flavor of the commercially available non-alcoholic beer-taste beverage A without the glycated proteins and a reference point of 0.5 for the rich flavor of the commercially available non-alcoholic beer-taste beverage A without the glycated proteins.

Evaluation was performed by varying the concentration of the glycated proteins added. Table 1 shows the results.

The sour flavor and the rich flavor were evaluated based on the following criteria.
0 points: no sour flavor or rich flavor at all
1 point: slight sour flavor or rich flavor
2 points: definite sour flavor or rich flavor
3 points: very strong sour flavor or rich flavor

Table 1 shows the glycated lysine content (nmol/mL) in the glycated proteins in the non-alcoholic beer-taste beverage.

**[Table 1]**

| | Concentration of glycated proteins added (µg/mL) | | | | | | |
|---|---|---|---|---|---|---|---|
| | Not added | 5 | 10 | 15 | 20 | 25 | 50 |
| Glycated lysine content in non-alcoholic beer-taste beveraqe (nmol/mL) | 0.0 | 4.7 | 9.4 | 14.1 | 18.8 | 23.5 | 47.0 |
| Intensity of sour flavor (point) | 3.0 | 2.84 | 2.64 | 2.48 | 2.30 | 2.20 | 1.96 |
| Intensity of rich flavor (point) | 0.5 | 0.5 | 0.52 | 0.56 | 0.64 | 0.68 | 0.84 |
| Δlntensity of sour flavor (point) | - | -0.16 | -0.36 | -0.52 | -0.70 | -0.80 | -1.04 |
| intensity of rich flavor (point) | - | 0 | 0.02 | 0.06 | 0.14 | 0.18 | 0.34 |

The results in Table 1 show that when the glycated proteins were added to the non-alcoholic beer-taste beverage to increase the glycated lysine content, the sour flavor of the non-alcoholic beer-taste beverage was reduced and the rich flavor thereof was enhanced.

### (Evaluation of sour flavor and rich flavor of commercially available non-alcoholic beer taste beverage A to which alcohol and glycated protein were added)

Ethanol was added to the commercially available non-alcoholic beer-taste beverage A such that the alcohol content was 0.999%. Further, the purified glycated proteins derived from beer were added and sensory evaluation of the sour flavor and rich flavor was performed.

The beverage A in this case still has an alcohol content of less than 1.0%, and thus is classified as a non-alcoholic beer-taste beverage.

The sensory evaluation was performed as in the case of evaluation of the commercially available non-alcoholic beer-taste beverage A to which the beer-derived glycated proteins were added.

Table 2 shows the glycated lysine content (nmol/mL) in the glycated proteins in the non-alcoholic beer-taste beverage.

**[Table 2]**

| | Concentration of glycated proteins added (µg/mL) | | |
|---|---|---|---|
| | Not added | 25 | 50 |
| Glycated lysine content in non-alcoholic beer-taste beverage (nmol/mL) | 0.0 | 23.5 | 47.0 |
| Intensity of sour flavor (point) | 2.7 | 2.1 | 1.72 |
| Intensity of rich flavor (point) | 0.6 | 0.8 | 0.94 |
| ΔIntensity of sour flavor (point) | - | -0.6 | -0.98 |
| ΔIntensity of rich flavor (point) | - | 0.2 | 0.34 |

The results in Table 2 show that when the glycated proteins were added to the non-alcoholic beer-taste beverage having an alcohol content of 0.999% to increase the glycated lysine content, the sour flavor of the non-alcoholic beer-taste beverage was reduced and the rich flavor thereof was enhanced.

### (Evaluation of sour flavor and rich flavor of commercially available non-alcoholic beer taste beverage B to which glycated proteins were added)

The purified glycated proteins derived from beer were added to the commercially available non-alcoholic beer-taste beverage B and sensory evaluation of the sour flavor and rich flavor was performed.

The commercially available non-alcoholic beer taste beverage B has an alcohol content of 0.00%.

The commercially available non-alcoholic beer taste beverage B has a purine concentration of less than 5.0 mg/100 mL.

In the sensory evaluation, five special panelists rated the commercially available non-alcoholic beer-taste beverage B containing the purified glycated proteins in 0.1-point increments, with a reference point of 3.0 for the sour flavor of the commercially available non-alcoholic beer-taste beverage A without the glycated proteins and a reference point of 0.5 for the rich flavor of the commercially available non-alcoholic beer-taste beverage A without the glycated proteins.

Evaluation was performed by varying the concentration of the glycated proteins added. Table 3 shows the results.

The sour flavor and rich flavor were evaluated based on the following criteria.
0 points: no sour flavor or rich flavor at all
1 point: slight sour flavor or rich flavor
2 points: definite sour flavor or rich flavor
3 points: very strong sour flavor or rich flavor
4 points: stronger sour flavor than commercially available non-alcoholic beer taste beverage A

Table 3 shows the glycated lysine content (nmol/mL) in the glycated proteins in the non-alcoholic beer-taste beverage.

**[Table 3]**

| | Concentration of glycated proteins added (µg/mL) | | |
|---|---|---|---|
| | Not added | 25 | 50 |
| Glycated lysine content in non-alcoholic beer-taste beverage (nmol/mL) | 0.0 | 23.5 | 47.0 |
| Intensity of sour flavor (point) | 3.6 | 3.2 | 2.8 |
| Intensity of rich flavor (point) | 0.4 | 0.5 | 0.6 |
| Δlntensity of sour flavor (point) | - | -0.4 | -0.8 |
| Δlntensity of rich flavor (point) | - | 0.1 | 0.2 |

The results in Table 3 show that when the glycated proteins were added to a different non-alcoholic beer-taste beverage to increase the glycated lysine content, the sour flavor of the non-alcoholic beer-taste beverage was reduced and the rich flavor of the non-alcoholic beer-taste beverage was enhanced.

### INDUSTRIAL APPLICABILITY

The present invention can provide a non-alcoholic beer-taste beverage with an enhanced rich flavor and a reduced sour flavor using a grain-derived raw material.

## Claims

1. A non-alcoholic beer-taste beverage having an alcohol content of less than 1.0%, the non-alcoholic beer-taste beverage comprising
a glycated protein which is a grain-derived protein, has a glycated amino group of lysine, and has a molecular weight of 35 to 50 kDa.

2. The non-alcoholic beer-taste beverage according to claim 1,
wherein the glycated protein has a glycated lysine content of 4.7 nmol/mL or more.

3. The non-alcoholic beer-taste beverage according to claim 1 or 2,
wherein the glycated protein has a glycated lysine content of 9.4 nmol/mL or more.

4. The non-alcoholic beer-taste beverage according to any one of claims 1 to 3,
wherein a sugar bound to the glycated amino group of the lysine is a monosaccharide or a disaccharide.

5. The non-alcoholic beer-taste beverage according to any one of claims 1 to 4,
wherein the grain is at least one selected from the group consisting of barley, wheat, corn, rice, and soybean.

6. The non-alcoholic beer-taste beverage according to any one of claims 1 to 5,
wherein the non-alcoholic beer-taste beverage contains no wort.

7. A rich flavor enhancing agent for non-alcoholic beer-taste beverages having an alcohol content of less than 1.0%, the agent comprising
a glycated protein which is a grain-derived protein, has a glycated amino group of lysine, and has a molecular weight of 35 to 50 kDa.

8. A rich flavor enhancing method for non-alcoholic beer-taste beverages, the method comprising
enhancing a rich flavor of a non-alcoholic beer-taste beverage by adding a glycated protein which is a grain-derived protein, has a glycated amino group of lysine, and has a molecular weight of 35 to 50 kDa to the non-alcoholic beer-taste beverage having an alcohol content of less than 1.0%.

9. A sour flavor reducing agent for non-alcoholic beer-taste beverages having an alcohol content of less than 1.0%, the agent comprising
a glycated protein which is a grain-derived protein, has a glycated amino group of lysine, and has a molecular weight of 35 to 50 kDa.

10. A sour flavor reducing method for non-alcoholic beer-taste beverages, the method comprising
reducing a sour flavor of a non-alcoholic beer-taste beverage by adding a glycated protein which is a grain-derived protein, has a glycated amino group of lysine, and has a molecular weight of 35 to 50 kDa to the non-alcoholic beer-taste beverage having an alcohol content of less than 1.0%.
